# EUROPÄISCHE PATENTANMELDUNG

(11) **EP 4 520 314 A2**
(43) Veröffentlichungstag der Anmeldung: **12.03.2025**
(21) Anmeldenummer: 24181610.7
(22) Anmeldetag: 12.06.2024
(51) Int. Cl.: A61K 8/41, A61K 8/42, A61K 8/60, A61K 8/73, A61Q 5/02, A61Q 5/12

(54) **REINIGUNGSGEL MIT BIOTENSIDEN**

(30) Priorität: 15.08.2023 DE 102023207846
(71) Anmelder: Henkel AG & Co. KGaA, 40589 Düsseldorf (DE)
(72) Erfinder: Westphal, Petra, 21629 Neu Wulmstorf (DE); Scheele, Soeren, 22765 Hamburg (DE); Mette, Manuela, 23923 Schönberg OT Kleinfeld (DE); Schroeder, Thomas, 22395 Hamburg (DE)

(57) **Zusammenfassung**

Kosmetische Reinigungszusammensetzungen in Form wässriger Gele, welche spezifische Mengen an Glycolipiden, auf natürlichen Polymeren basierenden Verdickungsmitteln sowie biologisch abbaubaren kationischen oder kationisierbaren Tensiden umfassen, eignen sich für die Reinigung und Pflege von Haaren.

## Beschreibung

Die Erfindung betrifft kosmetische, wässrige Reinigungsgele, umfassend Glycolipide, spezifische kationische Tenside sowie auf natürlichen Polymeren basierende Verdickungsmittel. Die Erfindung betrifft weiterhin die Verwendung der Reinigungsgele für die Reinigung und Pflege von Haaren.

Kosmetische Reinigungsmittel sind bekannt und werden im Handel in Flaschen oder in flexiblen Schläuchen verpackt angeboten. Ihre Viskosität wird im Allgemeinen derart eingestellt, dass sie durch leichten Druck aus einem flexiblen Schlauch oder durch leichten Druck auf eine Pumpe (die auf einer Pumpflasche befestigt ist) bequem entnommen werden können und nicht tropfen und/oder zwischen den Fingern verlaufen.

Üblicherweise werden kosmetische Reinigungsmittel zum Zeitpunkt des Auftragens mit Wasser verdünnt und anschließend auf der Anwendungsoberfläche verteilt oder einmassiert, wobei sich ein Schaum bildet.

Klassische kosmetische Reinigungsmittel umfassen in der Regel waschaktive Tenside mit ausgezeichnetem Reinigungs- und Schaumvermögen, welche zumeist petrochemischen Ursprungs sind oder auf Palmöl basieren.

Verbraucher legen bei der Auswahl kosmetischer Produkte jedoch immer mehr Wert auf Natürlichkeit, insbesondere dann, wenn sie diese täglich auf der Haut und/oder den Haaren anwenden. Daher sind klassische, gut schäumende Tenside petrochemischen Ursprungs, auf Palmöl und/oder Kokosöl basierende Tenside oder Sulfattenside - zum Teil auch aufgrund ihres Reizpotentials auf der Haut - regelmäßig Gegenstand kontroverser Diskussionen.

Es besteht daher ein echter Bedarf nach natürlicheren Körperhygieneprodukten, die einen möglichst hohen Anteil an Inhaltsstoffen aus umweltfreundlicheren, nachhaltigeren Quellen enthalten, und die eine gute Verträglichkeit aufweisen.

Der teilweise oder vollständige Ersatz der vorgenannten Tenside durch nachhaltigere Varianten kann allerdings zu Instabilitäten und Leistungsverlust der entsprechenden Reinigungsprodukte führen, wie in zahlreichen Versuchen gezeigt wurde. Als problematisch bei der Verwendung insbesondere höherer Mengen an Tensiden, die nicht petrochemischen Ursprungs oder aus Palmöl und/oder Kokosöl erhältlich sind - wie beispielsweise Glycolipiden - wird vornehmlich deren geringe Schaumbildungstendenz beschrieben (im Vergleich zu klassischen Tensiden wie etwa Sulfattensiden).

Daher wurden Glycolipiden enthaltenden Reinigungszusammensetzungen in der Vergangenheit oftmals weitere (anionische) Tenside hinzugefügt, um deren Schaumvermögen und ggfs. deren Reinigungsleistung zu steigern:
In DE 102015217501 wurden kosmetische Reinigungsmittel beschrieben, in denen Glycolipide mit Acyllactylaten kombiniert wurden, um das Schaumvermögen der kosmetischen Reinigungsmittel zu steigern.

Gemäß EP 2552389 führte die Kombination von Biotensiden mit anionischen Glycinat- oder Sulfosuccinattensiden sowie vorzugsweise mit weiteren schaumverstärkenden Tensiden zu kosmetischen Reinigungsmitteln mit hohem Reinigungs- und Schaumvermögen.

Als nicht-schäumende, Glycopilide enthaltende Zusammensetzungen wurden bislang meist micellare Zusammensetzungen für die Behandlung der Gesichtshaut und/oder zur Makeup-Entfernung beschrieben, beispielsweise in DE 102019202724.

Der vorliegenden Anmeldung lag die Aufgabe zugrunde, sehr gut hautverträgliche und pflegende kosmetische Reinigungsmittel mit hervorragender Reinigungsleistung bereitzustellen, die neben Glycolipiden keinen Gehalt weiterer waschaktiver Tenside erfordern. Die Reinigungsmittel sollen optimalerweise zu einem überwiegenden Teil auf Rohstoffen natürlichen Ursprungs basieren, worunter bevorzugt ein Gehalt von >90% (bezogen auf das gesamte Mittel) verstanden wird. Insbesondere sollen die Reinigungsmittel frei von Tensiden sein, die petrochemischen Ursprungs sind und/oder auf Palmöl und/oder Kokosöl basieren.

Es wurde nun gefunden, dass Reinigungsmittel auf der Basis spezifischer, in eine Gelmatrix eigebetteter Glycolipide, kationischer Tenside und natürlicher Verdickungsmittel diese Anforderungen in hohem Maße erfüllen.

Die erfindungsgemäßen Reinigungsgele weisen den Vorteil auf, dass für deren hervorragendes Reinigungsvermögen vollständig auf die Verwendung zusätzlicher waschaktiver (insbesondere anionischer, amphoterer, zwitterionischer oder nichtionischer) Tenside verzichtet werden kann. Die Reinigungsgele basieren überwiegend auf Rohstoffen natürlichen Ursprungs, sind gut hautverträglich und weisen außerdem sehr gute Haarpflegeeffekte auf.

Die erfindungsgemäßen Reinigungsgele schäumen nicht oder nur wenig, sind in ihrer Reinigungsleistung aber dennoch gleichwertig zu üblichen schäumenden, Sulfattenside enthaltenden Zusammensetzungen.

Weiterhin wurde gefunden, dass erfindungsgemäße Reinigungsgele besonders gut geeignet für die Behandlung gelockter und/oder krauser Haare sind, da ihre Applikation mit einer nur geringfügigen Verwirrung der Haare einhergeht und es somit nur zu einer äußerst geringen bis zu keiner Verkettung, Verklebung oder Verknotung kommt.

Ein erster Gegenstand der vorliegenden Erfindung ist daher eine kosmetische Reinigungszusammensetzung in Form eines wässrigen Gels, die (bezogen auf ihr Gesamtgewicht)
a) 0,5 - 15 Gew.-% mindestens eines Glycolipids
b) 0,5 - 20 Gew.-% mindestens eines auf natürlichen Polymeren basierenden Verdickungsmittels
c) 0,1 - 10 Gew.-% mindestens eines biologisch abbaubaren kationischen oder kationisierbaren Tensids enthält.

Unter Reinigungszusammensetzungen im Sinne der vorliegenden Erfindung sind prinzipiell alle für die Anwendung am menschlichen Körper geeigneten kosmetischen Reinigungsmittel zu verstehen. Besonders bevorzugt handelt es sich bei erfindungsgemäßen Reinigungszusammensetzungen um Haarshampoos und insbesondere um Haarshampoos in wässriger Gelform.

Unter dem Begriff "wässrig" wird im Sinne der vorliegenden Erfindung verstanden, dass die gelförmigen Reinigungszusammensetzungen vorzugsweise mindestens 60 Gew.-% Wasser, mehr bevorzugt mindestens 70 Gew.-%, besonders bevorzugt mindestens 80 Gew.-% und insbesondere mindestens 85 Gew.-% Wasser enthalten (bezogen auf das Gesamtgewicht der Reinigungsgele).

"Gele" können üblicherweise unterteilt werden in:
1. Gele mit lamellaren Ordnungsstrukturen einschließlich Gel-Mesophasen, z.B. Tensidgele, insbesondere Seifengele
2. Gele, die aus polymeren, dreidimensionalen, kovalent gebundenen Netzwerken, die ohne Zerstörung der Kovalenzbindungen in Lösungsmitteln unlöslich sind, aufgebaut sind
3. Gele aus polymeren Netzwerken mit lokalen Ordnungsstrukturen, die durch physikalische Kräfte zusammengehalten werden, z.B. Gelatine, Homo- und Copolymere von Acryl- und Methacrylsäure, Alginate, Carrageenan
4. Gele gebildet aus feinverteilten Bestandteilen, z.B. Niederschlägen, die i.a. eine große geometrische Anisotropie aufweisen, z.B. V₂O₅-Gele oder Gele, die sich durch Aggregation von Proteinen bilden.

Geeignete "Gele" im Sinne der nachfolgend beschriebenen Erfindung sind insbesondere solche, die unter Punkt 3. der obigen Auflistung fallen.

"Gele" im Sinne der vorliegenden Erfindung sollten vorzugsweise durch eine hohe Strukturviskosität gekennzeichnet sein, d.h., diese Zubereitungen erscheinen unter dem Einfluss geringer Scherkräfte, etwa des Eigengewichts, relativ hochviskos; lassen sich aber bei Anwendung höherer Scherkräfte, z.B. beim Verrühren sehr leicht bewegen.

Wässrige Reinigungsgele im Sinne der vorliegenden Erfindung weisen vorzugsweise eine Viskosität im Bereich von 3,000 bis 30,000 mPas, mehr bevorzugt von 5,000 bis 30,000 mPas, besonders bevorzugt 10,000 bis 27,500 mPas und insbesondere 12,500 bis 25,000 mPas auf, gemessen bei 20°C mit einem Brookfield Viskosimeter; Spindel 6; 20s; 20 Upm.

Der Begriff "natürlich" im Zusammenhang mit natürlichen Inhaltsstoffen für Kosmetika ist in der ISO 16128 festgelegt. Gemäß dieser Richtlinie kann man natürliche, kosmetische Inhaltsstoffe zusammenfassend als Stoffe beschreiben, die von Pflanzen, Tieren, Mineralien oder Mikroorganismen erhältlich sind, eingeschlossen sind auch solche Stoffe, die von den genannten Quellen durch physikalische Prozesse, Fermentationsprozesse (nur solche Fermentationsprozesse, die auch in der Natur ablaufen und die zu Produkten führen, die auch in der Natur entstehen) und andere Herstellungsmethoden ohne beabsichtigte chemische Modifikation erhalten werden. Die Mikroorganismen dürfen nur solche sein, die nicht gentechnisch modifiziert wurden.

Im Sinne der vorliegenden Erfindung sind Substanzen natürlichen Ursprungs, insbesondere natürliche Polymere oder Polymere natürlichen Ursprungs, Verbindungen, die von natürlichen Substanzen/Polymeren durch Modifizierungen abgeleitet sind. Damit sind auch chemische Modifizierungen umfasst. Der natürliche Grundcharakter der Substanzen/Polymere kann durch geeignete Modifizierung noch weiter optimiert werden.

Der Begriff der biologischen Abbaubarkeit beschreibt den Prozess des Abbaus von organischen Verbindungen durch Lebewesen, insbesondere Saprobionten. Im Idealfall entstehen anorganische Stoffe, wie beispielsweise CO₂, O₂ und Ammoniak; Verbindungen, die von Pflanzen und Mikroorganismen wieder für den Aufbau von organischen Verbindungen genutzt werden. Organische, chemische Verbindungen, die leicht biologisch abbaubar sind, sind nach OECD 301, für kosmetische Zusammensetzungen meist nach OECD 301 B klassifiziert. Diese Substanzen oder Zusammensetzungen können rasch und vollständig abgebaut werden.

Organische, chemische Verbindungen, die nach OECD 302 klassifiziert sind, sind zwar eingeschränkt, jedoch grundsätzlich biologisch abbaubar.

Als ersten wesentlichen Inhaltsstoff enthalten die erfindungsgemäßen Reinigungsgele 0,5 - 15 Gew.-% mindestens eines Glycolipids. Bevorzugt sind Einsatzmengen des mindestens einen Glycolipids a) von 0,75 - 12,5 Gew.-%, mehr bevorzugt von 1 - 10 Gew.-% und insbesondere von 1 - 7,5 Gew.-%, bezogen auf das Gesamtgewicht des erfindungsgemäßen Reinigungsgels.

Glycolipide gehören zu den sogenannten Biotensiden, d.h. Substanzen, die von Mikroorganismen gebildet und oftmals auch aus der Zelle ausgeschleust werden. Biotenside sind wie die klassischen Tenside oberflächenaktive Substanzen, welche die Oberflächenspannung von Flüssigkeiten verringern und dadurch die Vermischung von wässrigen (hydrophilen) und wasserabstoßenden (hydrophoben) Phasen fördern. Biotenside sind unter milden Produktionsbedingungen, die einen geringen Energieverbrauch bedingen, herstellbar. Sie sind in der Regel gut biologisch abbaubar und ihre Umweltverträglichkeit ist sehr hoch. Außerdem sind sie nicht toxisch und bei ihrer Produktion fallen auch keine toxischen Nebenprodukte an. Als Rohstoffe für deren mikrobielle Herstellung werden Kohlenhydrate, insbesondere Zucker wie z.B. Glucose und/oder lipophile KohlenstoffQuellen wie Fette, Öle, Partialglyceride, Fettsäuren, Fettalkohole, langkettige gesättigte oder ungesättigte Kohlenwasserstoffe eingesetzt. Bevorzugt sind durch Fermentation hergestellte Biotenside.

Geeignete Glycolipide im Sinne der vorliegenden Erfindung sind Verbindungen, in denen eine oder mehrere Monosaccharideinheiten glycosidisch mit einem Lipidanteil verbunden sind. Beispiele für besonders geeignete Glycolipide im Sinne der vorliegenden Erfindung sind Rhamnolipide, Sophorolipide, Mannosylerythritollipide und Trehaloselipide, Cellobiolipide, Saponine oder Mischungen davon.

Für die Verwendung in der vorliegenden Erfindung haben sich insbesondere Rhamnolipide als besonders bevorzugt erwiesen, denn sie lassen sich problemlos mit erfindungsgemäßen kationischen oder kationisierbaren Tensiden und den spezifischen Verdickungsmitteln b) in eine stabile Gelmatrix einbinden und weisen ein hervorragendes Reinigungspotential auf.

Rhamnolipide erhält man von Bakterien der Gattung Pseudomonas, insbesondere von Pseudomonas aeruginosa, bevorzugt bei Wachstum auf hydrophoben Substraten wie n-Alkanen oder Pflanzenölen. Weitere Glycolipide, etwa Glucoselipide, Cellobioselipide oder Trehaloselipide, werden von wieder anderen Mikroorganismen auf unterschiedlichen Substraten produziert. Weiterhin sind erfindungsgemäß Mannosylerythritollipide bevorzugte Glycolipid-Biotenside; diese werden von Bakterien der Pseudozyma sp., Candida antarctica und Ustilago sp. produziert.

Rhamnolipide lassen sich durch die folgende Strukturformel beschreiben: 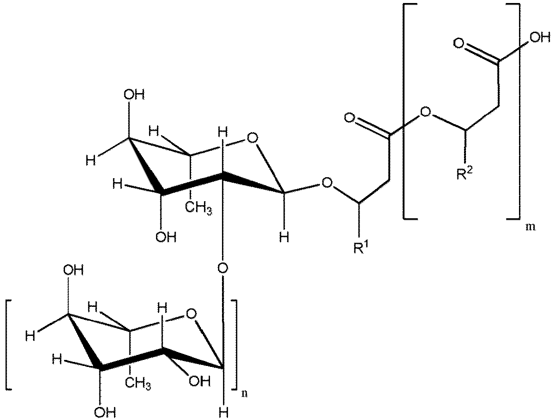 wobei
- m 2, 1 oder 0 ist,
- n 1 oder 0 ist,
- R¹ und R² unabhängig voneinander ein gleicher oder verschiedener organischer Rest mit 2 bis 24, bevorzugt 5 bis 13 Kohlenstoffatomen ist, insbesondere ein substituierter oder unsubstituierter, verzweigter oder unverzweigter Alkylrest, der auch ungesättigt sein kann, wobei der Alkylrest bevorzugt ein linearer gesättigter Alkylrest mit 8 bis 12 Kohlenstoffatomen ist, weiter bevorzugt ein Nonyl- oder Decylrest oder ein Gemisch davon.

Salze dieser Verbindungen sind erfindungsgemäß ebenfalls umfasst.

Unter dem Begriff "di-Rhamnolipid" in der vorliegenden Erfindung werden Verbindungen der obigen Formel oder deren Salze verstanden, bei denen n 1 ist.

Entsprechend werden unter "mono-Rhamnolipid" in der vorliegenden Erfindung Verbindungen der allgemeinen Formel oder deren Salze verstanden, bei denen n 0 ist.

Als Beispiel für ein besonders geeignetes Rhamnolipid kann das unter der Handelsbezeichnung Rheance One^{®} von der Firma Evonik erhältliche Produkt genannt werden.

Sophorolipide werden fermentativ unter Verwendung von Hefen wie Candida bombicola (auch als Torulopsis bombicola bekannt), Yarrowia lipolytica, Candida apicola (Torulopsis apicola) und Candida bogoriensis produziert, indem man diese auf Zuckern, Kohlenwasserstoffen, Pflanzenölen oder Mischungen davon wachsen lässt.

Sophorolipide weisen die unten angegebenen Formeln (1) (Lactonform) und (2) (freie Säure) auf, wobei die beiden Formen üblicherweise im Gemisch vorliegen. 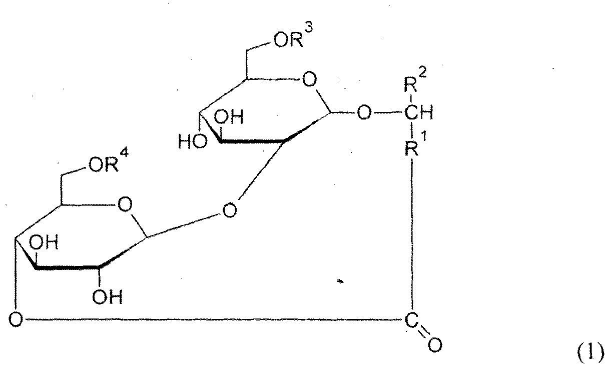 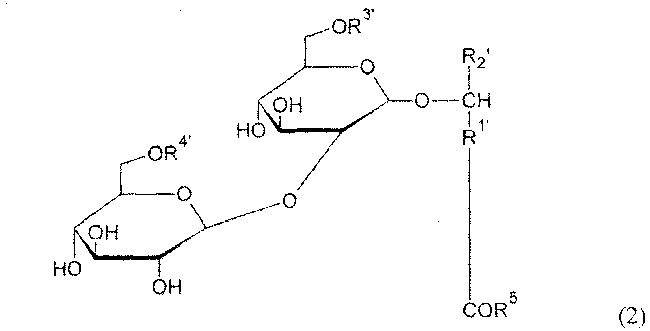 wobei
- R¹ und R¹' unabhängig voneinander gesättigte Kohlenwasserstoffketten oder einfach oder mehrfach, insbesondere einfach, ungesättigte Kohlenwasserstoffketten mit 8 bis 20, insbesondere 12 bis 18 Kohlenstoffatomen, weiter bevorzugt 14 bis 18 Kohlenstoffatomen darstellen, die linear oder verzweigt sein können und ein oder mehrere Hydroxygruppen aufweisen können,
- R² und R²' unabhängig voneinander ein Wasserstoffatom oder eine gesättigter Alkylrest oder ein einfach oder mehrfach, insbesondere einfach, ungesättigter Alkylrest mit 1 bis 9 Kohlenstoffatomen darstellen, bevorzugter 1 bis 4 Kohlenstoffatomen, die linear oder verzweigt sein können und ein oder mehrere Hydroxygruppen aufweisen können, und
- R³, R³', R⁴ und R⁴'unabhängig voneinander ein Wasserstoffatom oder eine Acetylgruppe darstellen.

Bevorzugt sind solche Sophorolipide, bei denen R¹ und R¹' eine einfach ungesättigte, lineare Kohlenwasserstoffkette mit 15 Kohlenstoffatomen sind. Bevorzugt ist weiterhin, dass R² und R²' eine Methylgruppe oder ein Wasserstoffatom darstellen, noch bevorzugter jeweils eine Methylgruppe.

Erfindungsgemäß bevorzugt sind Sophorolipide, bei denen die Säureform und die Lactonform im Gemisch vorliegen, wobei bevorzugt etwa 20 bis etwa 60 Gew.-% des Sophorolipids als Säureform vorliegt und der Rest des Sophorolipids in der Lactonform vorliegt.

Insbesondere sind Sophorolipide bevorzugt, bei denen Verbindungen der obigen Formeln (1) und (2) in einem Gemisch vorliegen, wobei R¹ und R^{1'}eine einfach ungesättigte, lineare Kohlenwasserstoffkette mit 14 bis 18 Kohlenstoffatomen, noch bevorzugter 15 Kohlenstoffatomen, sind, R³ und R⁴ eine Acetylgruppe darstellen, R^{3'} und R^{4'} ein Wasserstoffatom darstellen und R² und R^{2'} eine Methylgruppe darstellen, und wobei etwa 20 bis 60 Gew.-% der Sophorolipide in der Säureform vorliegen.

Derartige Sophorolipide sind im Handel erhältlich, beispielsweise unter der Bezeichnung Sopholiance S von der Firma Soliance. Genauer ist das unter dem Handelsnamen Sopholiance S von der Firma Soliance erhältliche Sophorolipid eine etwa 60 Gew.-% Sophorolipid-Lösung und wird beispielsweise durch Fermentierung von Candida bombicola auf Rapsöl-Methylester und Glucose gewonnen (INCI: Candida bombicola / glucose/ methyl rapeseed ferment (and) Water). Sopholiance S ist ein erfindungsgemäß bevorzugtes Sophorolipid.

Bei Soliance S liegt zu etwa 20 Gew.-% die freie Säureform vor, im Gemisch mit der Lactonform.

Mannosylerythritollipide sind Glycolipide der folgenden allgemeinen Formel: 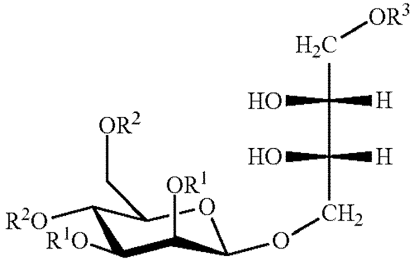 worin die R¹ unabhängig voneinander Fettsäureacylgruppen mit 4 bis 24 Kohlenstoffatomen, bevorzugt 8 bis12 Kohlenstoffatomen, darstellen, die R² unabhängig voneinander ein Wasserstoffatom oder eine Acetylgruppe darstellen und R³ ein Wasserstoffatom oder eine Fettsäureacylgruppe mit 2 bis 24 Kohlenstoffatomen darstellt. Ein erfindungsgemäß geeignetes Mannosyerythritollipid ist im Handel unter der Bezeichnung Ceramela-B (Toyobo) (INCI: Pseudozyma Tsukubaensis/Olive Oil/Glycerin/Soy Protein Ferment) erhältlich.

Saponine sind natürliche Alkylglucosid-Tenside, die aus Molekülen mit einer oder mehreren linearen oder verzweigten hydrophilen Glycosideinheiten bestehen, die an ein lipophiles Triterpen oder Steroid-Aglykon (Sapogenin) gebunden sind. Bevorzugt können Triterpenglycoside sein. Saponine kommen beispielsweise in der Seifenkrautpflanze (Gattung Saponaria) vor, deren Wurzel früher als Seife verwendet wurde.

Eine andere Saponinquelle bietet der Seifenrindenbaum. Die innere Rinde des Seifenrindenbaums kann zu Pulver zerkleinert und als Ersatz für Seife verwendet werden, da sie aufgrund eines Gehalts an Saponin mit Wasser einen Schaum bildet.

Saponine sind amphiphatische Glycoside, die beim Schütteln in wässrigen Lösungen seifenähnlichen Schaum erzeugen können.

In einer bevorzugten Ausführungsform enthalten erfindungsgemäße Reinigungsgele als mindestens ein Glycolipid a) mindestens ein Rhamnolipid in den zuvor genannten Mengen.

Als zweiten wesentlichen Inhaltsstoff enthalten die erfindungsgemäßen Reinigungsgele 0,5 - 20 Gew.-% mindestens eines auf natürlichen Polymeren basierenden Verdickungsmittels b). Bevorzugt sind Einsatzmengen des mindestens einen Verdickungsmittels b) von 1 - 20 Gew.-%, mehr bevorzugt von 1,5 - 17,5 Gew.-% und insbesondere von 2 - 15 Gew.-%, bezogen auf das Gesamtgewicht des erfindungsgemäßen Reinigungsgels.

Geeignete auf natürlichen Polymeren basierende Verdickungsmittel b) können vorzugsweise ausgewählt sein aus den folgenden Gruppen der sogenannten Biopolymere und/oder Derivaten von Biopolymeren:
1) Galactomanne wie beispielsweise Guar Gum, Tara Gum, Johannisbrotkernmehl und/oder Cassia Gum
2) Biosaccharidgumme wie Xanthan Gum, Gummi arabicum, Ghatti Gum, Karaya Gum, Tragant Gum, Carrageenan Gum, Leinsamengummi, Agar-Agar, Pektine und/oder Alginate
3) Stärke-Fraktionen und/oder Derivate von Stärke und/oder Amylose, Amylopektin und/oder Dextrin
4) Cellulose und/oder Cellulosederivate wie Alkylcellulose (Methylcellulose, Ethylcellulose, Propylcellulose), Carboxyalkylcellulose (Carboxymethylcellulose, Carboxyethylcellulose) und/oder Hydroxyalkylcellulose (Hydroxymethylcellulose, Hydroxyethylcellulose, Hydroxypropylcellulose, Hydroxypropyl Methylcellulose)
5) Gelatine
6) Tone und/oder Schichtsilikate.

Bevorzugt sind Verdickungsmittel b) aus den zuvor genannten Gruppen 1) - 4): Gumme, Alginate, Pektine, Stärke oder Stärkederivate, Cellulose oder Cellulosederivate, Dextrine oder Mischungen davon.

Ganz besonders bevorzugt sind Stärke und/oder Stärkederivate, denn mit ihnen lässt sich eine für die Zwecke der vorliegenden Erfindung besonders stabile Gelmatrix bereitstellen, die für die Stabilisierung der Komponente(n) a) und c) sowie für die Lagerstabilität der erfindungsgemäßen Reinigungsgele erforderlich ist.

Unter geeigneten Stärken im Sinne der vorliegenden Erfindung werden vorzugsweise Stärken aus Kartoffeln, Mais, Reis, Erbsen, Eicheln, Kastanien, Gerste, Weizen, Bananen, Sago, Hirse, Sorghum, Hafer, Gerste, Roggen, Bohnen, Batate, Maranta oder Maniok verstanden.

Besonders bevorzugt sind Tapiokastärke, Kartoffelstärke, Maisstärke und/oder oder Reisstärke.

Unter Stärkederivaten sind vorzugsweise hydrophob modifizierte Stärkeverbindungen aus Mais, Weizen, Reis, Tapioka, Kartoffeln und/oder Sago verstanden, die hydrophob modifiziert wurden. Unter "hydrophober Modifizierung" wird im Sinne der vorliegenden Erfindung die chemische Vernetzung, beispielsweise durch ionische Vernetzung, mit Calcium, Aluminium und/oder Polyphosphaten - vorzugsweise mit Polyphosphaten - und/oder die chemische Modifizierung durch die Einführung hydrophober Gruppen verstanden.

Unter "hydrophoben Gruppen" werden vorzugsweise nichtionische Reste wie beispielsweise Hydroxyalkylgruppen verstanden, wobei "Hydroxyalkylgruppen" bevorzugt Hydroxyethyl-, Hydroxypropyl- und/oder Hydroxybutylgruppen bedeuten.

Besonders bevorzugte hydrophob modifizierte Stärkeverbindungen im Sinne der vorliegenden Erfindung sind vernetzt und tragen hydrophobe, nichtionische Substituenten, besonders bevorzugt Hydroxypropylgruppen.

Ganz besonders bevorzugt sind die unter den INCI-Bezeichnungen Hydroxyethyl Starch Phosphate und Hydroxypropyl Starch Phosphate bekannten hydrophob modifizierten Stärkeverbindungen. Insbesondere bevorzugt ist Hydroxypropyl Starch Phosphate.

Entsprechende Produkte sind bekannt und im Handel erhältlich, beispielsweise unter den Bezeichnungen Structure^{®} XL, Structure^{®} ZEA oder Stardesign^{®} Care.

Hydroxypropyl Starch Phosphate ist ein im Sinne der vorliegenden Erfindung ganz besonders geeignetes Verdickungsmittel b), denn es lässt sich ohne großen Energieaufwand und/oder Stabilitätsprobleme in einem Kaltprozess mit den Komponenten a) und c) kombinieren und bildet höherviskose Gele, die in einem breiten Temperaturbereich stabil bleiben.

In einer bevorzugten Ausführungsform enthalten die erfindungsgemäßen Reinigungsgele als Verdickungsmittel b) daher unter der INCI-Bezeichnung Hydroxypropyl Starch Phosphate bekannte Verbindungen.

Als dritten wesentlichen Inhaltsstoff enthalten die erfindungsgemäßen Reinigungsgele 0,1 - 10 Gew.-% mindestens eines biologisch abbaubaren kationischen oder kationisierbaren Tensids c).

Unter kationischen oder kationisierbaren Tensiden werden Tenside mit ausschließlich kationischen Ladungen verstanden; die beispielsweise quartäre Ammoniumgruppen oder Amingruppen enthalten, welche kationisiert werden können.

Geeignete kationische oder kationisierbare Tenside c) im Sinne der vorliegenden Erfindung können ausgewählt sein aus
1) sogenannten Amidoaminen der nachfolgenden Formel (I) 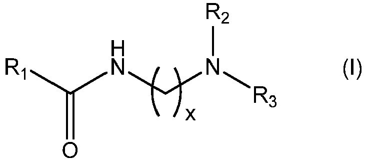 worin
   - R₁ für einen gesättigten oder ungesättigten, verzweigten oder unverzweigten Alk(en)ylrest mit 18 bis 30 Kohlenstoffatomen steht,
   - R₂ und R₃ unabhängig voneinander für Wasserstoff oder für eine C₁-C₄-Alkylgruppe stehen, und
   - X für eine ganze Zahl von 2 bis 6 steht.

Amidoamine der Formel (I) können in der Wirkstoffmischung der erfindungsgemäßen Reinigungsgele ein ausgesprochen weiches, geschmeidiges Haargefühl bewirken, ohne dass die Haare beschwert werden.

Besonders geeignet sind Amidoamine gemäß Formel (I), worin
- R₁ für einen Alkenylrest mit 18 bis 24 Kohlenstoffatomen steht,
- R₂ und R₃ für Methylgruppen stehen, und
- x für die Zahlen 2, 3 oder 4 steht.

Insbesondere bevorzugt für die Verwendung in den erfindungsgemäßen Reinigungsgelen sind Amidoamine gemäß Formel (I), die aus unter den INCI-Bezeichnungen Brassicamidopropyl Dimethylamin, Stearamidopropyl Dimethylamin, Behenamidopropyl Dimethylamin, Cannabisamidopropyl Dimethylamine bekannten Verbindungen sowie deren Mischungen ausgewählt sind.

2) sogenannten Esterquats der nachfolgenden Formel (II) 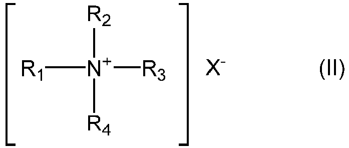 worin
- R₁, R₂ gleich oder verschieden sind und für einen C₁-C₄-Alkylrest stehen,
- R₃, R₄ gleich oder verschieden sind und für die Gruppierung -CH₂-CH(R₅)-OR₆ stehen, in der
- R₅ für einen C₁-C₆-Alkylrest steht,
- R₆ für einen Acylrest einer mindestens einfach ungesättigten Carbonsäure mit einer C-Kettenlänge von 18 bis 24 Kohlenstoffatomen oder für den Acylrest der Isostearinsäure steht, und
- X⁻ für ein Halogenid-, Methylsulfat-, Ethylsulfat-, Maleat-, Fumarat-, Oxalat-, Tartrat-, Citrat-, Lactat- oder Acetation steht.

Esterquats gemäß Formel (II) können in der Wirkstoffmischung der erfindungsgemäßen Reinigungsgele die haarkonditionierende Wirkung - insbesondere den Haargriff - verstärken.

Besonders geeignete Esterquats gemäß Formel (II) sind die unter den INCI-Bezeichnungen Distearoylethyl Hydroxyethylmonium Methosulfate, Distearoylethyl Dimonium Chloride, Distearoyl Dimonium Chloride, Distearoyl Dimonium Methosulfate, Bis-(Isostearoyl/Oleyl Isopropyl)Dimonium Methosulfate, Bis-(Isostearoyl/Oleyl Isopropyl)Dimonium Chloride bekannten Verbindungen sowie deren Mischungen.

Zur Verstärkung der haarpflegenden Eigenschaften kann es für einige Ausführungsformen zudem von Vorteil sein, wenn Kombinationen von zuvor genannten Amidoamine und Esterquats in den erfindungsgemäßen Reinigungsgelen eingesetzt werden.

Eine besonders bevorzugte Ausführungsform der erfindungsgemäßen Reinigungsgele ist dadurch gekennzeichnet, dass sie - bezogen auf ihr Gesamtgewicht -
a) 0,5 - 15 Gew.-% mindestens eines Rhamnolipids
b) 0,5 - 20 Gew.-% mindestens einer Stärke oder Stärkederivats,
c) 0,1 - 10 Gew.-% mindestens eines Esterquats und/oder Amidoamins enthalten.

Innerhalb dieser Ausführungsform sind erfindungsgemäße Reinigungsgele ganz besonders bevorzugt, die - bezogen auf ihr Gesamtgewicht -
a) 0,5 - 15 Gew.-% mindestens eines Rhamnolipids
b) 0,5 - 20 Gew.-% Hydroxypropyl Starch Phosphate,
c) 0,1 - 10 Gew.-% Brassicamidopropyl Dimethylamin, Stearamidopropyl Dimethylamin, Behenamidopropyl Dimethylamin Distearoylethyl Hydroxyethylmonium Methosulfate, Distearoylethyl Dimonium Chloride, Distearoyl Dimonium Chloride, Distearoyl Dimonium Methosulfate, Bis-(Isostearoyl/Oleyl Isopropyl)Dimonium Methosulfate, Bis-(Isostearoyl/Oleyl Isopropyl)Dimonium Chloride sowie Mischungen davon enthalten.

Neben den zuvor genannten wesentlichen Inhaltsstoffen a) - c) können die erfindungsgemäßen Reinigungsgele weitere fakultative Wirkstoffe enthalten - vorzugsweise Wirkstoffe, mit denen die Haarpflegeeffekte weiter verstärkt oder unterstützt werden können.

Bevorzugte fakultative Wirkstoffe können vorzugsweise ausgewählt sein aus kosmetischen Ölen und/oder feuchtigkeitsspendenden Komponenten.

Unter geeigneten kosmetischen Ölen werden im Sinne der vorliegenden Erfindung Öle aus der Gruppe der Verbindungen Polycitronellol, Polycitronellolacetat, Triolein oder Mischungen davon einerseits und/oder Pflanzenölen andererseits verstanden.

Sie können in den erfindungsgemäßen Reinigungsgelen eingesetzt werden, um den weichen Haargriff, das Haarvolumen und den Haarglanz weiter zu verbessern.

Öle aus der Gruppe der Verbindungen Polycitronellol, Polycitronellolacetat, Triolein oder Mischungen davon können in den erfindungsgemäßen Reinigungsgelen - bezogen auf deren Gesamtgewicht - vorzugsweise in Mengen von 0,01 - 5 Gew.-% eingesetzt werden.

Unter erfindungsgemäß geeigneten Pflanzenölen werden vorzugsweise Abyssinianöl, Amaranthsamenöl, Aprikosenkernöl, Arganöl, Avocadoöl, Babassuöl, Bambusöl, Baumwollsaatöl, Borretschsamenöl, Camelinaöl, Cranberrykernöl, Distelöl, Erdnussöl, Granatapfelkernöl, Grapefruitkernöl, Hanföl, Haselnussöl, Holundersamenöl, Johannesbeersamenöl, Jojobaöl, Kakaobutter, Kaktusfeigenöl, Krambesamenöl, Kürbiskernöl, Leinsamenöl, Macadamianussöl, Maiskeimöl, Malvenöl, Mandelöl, Mangokernöl, Marulaöl, Nachtkerzenöl, Olivenöl, Orangenöl, Palmöl, Pfirsichkernöl, Quinoaöl, Rambutanöl, Rapsöl, Reiskleieöl, Rizinusöl, Safloröl, Sanddornfruchtfleischöl, Sanddornkernöl, Sasanquanaöl, Sesamöl, Sheabutter, Sojaöl, Sonnenblumenöl, Teebaumöl, Traubenkernöl, Tsubakiöl, Walnussöl, Weizenkeimöl, Wiesenschaumkrautöl, Wildrosenöl, den flüssigen Anteilen von (süßem) Kokosöl oder Mischungen davon verstanden.

Pflanzenöle können in den erfindungsgemäßen Reinigungsgelen - bezogen auf deren Gesamtgewicht - vorzugsweise in Mengen von 0,01 - 5 Gew.-% eingesetzt werden.

In einer bevorzugten Ausführungsform enthalten erfindungsgemäße Reinigungsgele mindestens ein Öl aus der Gruppe der Verbindungen Polycitronellol, Polycitronellolacetat, Triolein und mindestens ein Pflanzenöl in einer Gesamtmenge von 0,01 - 5 Gew.-% (bezogen auf das Gesamtgewicht der Reinigungsgele).

Unter geeigneten feuchtigkeitsspendenden Komponenten, welche den Haaren und/oder der Kopfhaut vorteilhafte Eigenschaften verleihen, sind beipsielsweise Diole und Polyole wie Propylenglycol, Butylenglycol, Methylpropandiol, Ethylhexylglycerin, Glycerin, Diglycerin, Xylitol, Sorbitol, weiterhin Harnstoff und/oder Hyaluronsäure sowie deren physiologisch verträgliche Salze und Derivate, Ceramide, Phytosterole, Pflanzenextrakte wie Algen- oder Aloe Vera-Extrakt, Säuren wie Aminosäuren, Pyrrolidoncarbonsäure, Milchsäure bzw. deren physiologisch verträglichen Salze und/oder natürliche Betainverbindungen zu verstehen.

Bevorzugte feuchtigkeitsspendende Komponenten für die Verwendung in den erfindungsgemäßen Reinigungsgelen sind ausgewählt aus Propylenglycol, Butylenglycol, Glycerin, Sorbitol, Xylitol, Aloe Vera Gel, Hyaluronsäure(derivaten) oder Mischungen davon. Insbesondere bevorzugt ist Glycerin. Eine oder mehrere der zuvor genannten feuchtigkeitsspendenden Komponenten können in den erfindungsgemäßen Reinigungsgelen in Gewichtsanteilen von 0,1 - 10 Gew.-% am Gesamtgewicht der Reinigungsgele eingesetzt werden.

In einer bevorzugten Ausführungsform enthalten erfindungsgemäße Reinigungsgele mindestens eines der zuvor genannten Öle sowie mindestens eine feuchtigkeitsspendende Komponente in den zuvor genannten Mengen.

Innerhalb dieser Ausführungsform ist es ganz besonders bevorzugt, wenn die erfindungsgemäßen Reinigungsgele - bezogen auf ihr Gesamtgewicht -
> 0,01 - 2 Gew.-% Citronellol und/oder mindestens ein Pflanzenöl und
> 0,1 - 5 Gew.-% Glycerin enthalten.

Für die Herstellung, Viskositätseinstellung und/oder die Stabilisierung der erfindungsgemäßen Reinigungsgele ist es nicht erforderlich, dass Ihnen zusätzlich synthetische (polymere) Verdickungsmittel und/oder alkoxylierte Emulgatoren und/oder jedwede weiteren Inhalts- oder Wirkstoffe hinzugefügt werden, die petrochemischen Ursprungs sind und/oder auf Palmöl und/oder Kokosöl basieren.

In einer weiteren bevorzugten Ausführungsform sind erfindungsgemäße Reinigungsgele deshalb im Wesentlichen frei von alkoxylierten Emulgatoren oder Tensiden.

In einer weiteren bevorzugten Ausführungsform sind erfindungsgemäße Reinigungsgele deshalb im Wesentlichen frei von synthetischen polymeren Verdickungsmitteln.

In einer weiteren bevorzugten Ausführungsform sind erfindungsgemäße Reinigungsgele zudem im Wesentlichen frei von Inhalts- oder Wirkstoffen, die petrochemischen Ursprungs sind und/oder auf Palmöl und/oder Kokosöl basieren.

Unter "im Wesentlichen frei" wird ein Gehalt von maximal 0,5 Gew.-%, vorzugsweise maximal 0,25 Gew.-% und insbesondere 0,1 Gew.-% (bezogen auf das gesamte Gewicht des Mittels) verstanden, wobei sich der jeweilige Gehalt auf frei zugesetzte Anteile an alkoxylierten Emulgatoren oder Tensiden sowie auf frei zugesetzte synthetische polymere Verdickungsmittel und/oder auf weitere Inhalts- oder Wirkstoffe bezieht, die petrochemischen Ursprungs sind und/oder auf Palmöl und/oder Kokosöl basieren. Handelsproduktmischungen, in denen die o.g. Inhaltsstoffe unter Umständen in untergeordneten Mengen enthalten sein können, sind davon nicht umfasst.

Neben den zwingenden Bestandteilen und den zuvor genannten fakultativen bevorzugten Wirkstoffen können die erfindungsgemäßen Reinigungsgele weitere Wirk-, Hilfs- und Zusatzstoffe enthalten, die aus einem oder mehreren Bestandteilen aus einer oder mehreren der folgenden Gruppen ausgewählt sein können:
> Proteinhydrolysaten pflanzlichen oder tierischen Ursprungs, beispielsweise Elastin-, Kollagen-, Keratin-, Seiden- und Milcheiweiß-Proteinhydrolysaten, die auch in Form von Salzen vorliegen können; Soja-, Mandel-, Erbsen-, Moringa-, Kartoffel- und Weizenproteinhydrolysaten; Kollagenhydrolysaten von Fischen oder Algen sowie Proteinhydrolysaten von Muscheln bzw. Perlenhydrolysaten. Die Proteinhydrolysate können in den erfindungsgemäßen Reinigungsgelen in einem Gewichtsanteil von 0,001 Gew.-% bis 20 Gew.-%, mehr bevorzugt von 0,05 Gew.-% bis 15 Gew.-% und ganz besonders bevorzugt von 0,10 Gew.-% bis 5 Gew.-% am Gesamtgewicht des Mittels enthalten sein.
> Vitaminen, Provitaminen und/oder Vitaminvorstufen aus den Gruppen A, B, C, E, F und H, insbesondere aus den Gruppen A, B, E und H. Panthenol, Patolacton, Pyridoxin sowie Nicotinsäureamid und Biotin sind besonders bevorzugt. Vitamine, Provitamine und/oder Vitaminvorstufe der zuvor genannten Gruppen können in den erfindungsgemäßen Reinigungsgelen bevorzugt in einem Gewichtsanteil von jeweils 0,0001 Gew.-% bis 1,0 Gew.-%, mehr bevorzugt von 0,0005 bis 0,75 Gew.-% und insbesondere von 0,001 bis 0,5 Gew.-% am Gesamtgewicht des Reinigungsgels eingesetzt werden.
> Betainen wie Carnitin, Histidin, Cholin, Betain (Trimethylglycin) und/oder Taurin sowie deren physiologisch verträglichen Salzen und/oder Derivaten. Die zuvor genannten Betaine können in den erfindungsgemäßen Reinigungsgelen bevorzugt in einem Gewichtsanteil von jeweils 0,0001 Gew.-% bis 5,0 Gew.-%, mehr bevorzugt von 0,0005 bis 3,0 Gew.-% und insbesondere von 0,001 bis 1,0 Gew.-% am Gesamtgewicht des Reinigungsgels eingesetzt werden.
> Biochinonen wie Ubichinonen und/oder Plastochinonen - insbesondere Coenzym Q10 - in einem bevorzugten Gewichtsanteil von 0,001 Gew.-% bis 2,5 Gew.-%, mehr bevorzugt 0,0025 Gew.-% bis 1 Gew.-% und insbesondere 0,01 Gew.-% bis 0,1 Gew.-% am Gesamtgewicht des Reinigungsgels.
> Purinen - insbesondere Xanthin, Coffein, Theobromin oder Theophyllin - in einem bevorzugten Gewichtsanteil von 0,001 Gew.-% bis 1,0 Gew.-%, mehr bevorzugt 0,0025 Gew.-% bis 0,5 Gew.-% und insbesondere 0,01 Gew.-% bis 0,1 Gew.-% am Gesamtgewicht des Reinigungsgels.
> Ectoin in einem bevorzugten Gewichtsanteil von 0,001 Gew.-% bis 0,5 Gew.-%, mehr bevorzugt 0,0025 Gew.-% bis 0,3 Gew.-% und insbesondere 0,01 Gew.-% bis 0,1 Gew.-% am Gesamtgewicht des Reinigungsgels.
Ferner aus
> Pflanzenextrakten
> Antischuppenwirkstoffen aus natürlichen Quellen wie beispielsweise Extrakten aus Arnika, Birke, Klettenwurzel, Bartflechte, Pappel, Brennessen, Walnußschalen und/oder Propandiol Caprylate
> natürlichen Pigmenten und/oder Farbstoffen zur Anfärbung der Reinigungsgele
> Parfümölen, Duftstoffen und Riechstoffen
> Konservierungsmitteln
> Mitteln zur Einstellung des pH-Wertes wie beispielsweise NaOH und/oder Säuren wie Zitronensäure und/oder Milchsäure.

Bezüglich weiterer fakultativer Komponenten sowie die eingesetzten Mengen dieser Komponenten wird ausdrücklich auf die dem Fachmann bekannten einschlägigen Handbücher verwiesen.

Die erfindungsgemäßen Reinigungsgele können in einem breiteren pH-Bereich zur Verfügung gestellt werden und weisen eine ausgezeichnete Stabilität in diesem pH-Bereich auf. Vorzugsweise weisen die erfindungsgemäßen Reinigungsgele einen pH-Wert im Bereich von 4,0 bis 6,0, besonders bevorzugt 4,5 bis 6,0 und insbesondere 5,0 ± 0,5 auf.

Ebenso Gegenstand der Erfindung ist die Verwendung der erfindungsgemäßen kosmetischen Reinigungszusammensetzung für die Reinigung und Pflege von Haaren, insbesondere zur Verbesserung der Entwirrbarkeits-, Kämmbarkeits- und der statischen Eigenschaften von Haaren sowie zur Verbesserung des Haarvolumens und der Haarfülle.

### Beispiele:

Die nachfolgenden Beispiele sollen die vorliegende Erfindung verdeutlichen, ohne sie einzuschränken. Die Mengenangaben sind in Gew.-% und beziehen sich auf den Aktivgehalt, sofern nicht anders angegeben.

| | **1** | **2** | **3** | **4** |
|---|---|---|---|---|
| Glycolipid a) | 0,5-15 | | | |
| Rhamnolipid | | 0,5-15 | 0,5-15 | |
| Rheance One^{®*1} | | | | 8 |
| Verdickungsmittel b) | 0,5-20 | | | |
| Gumme, Alginate, Pektine, Stärke, Stärkederivate, Cellulose, Cellulosederivate und/oder Dextrine | | 0,5-20 | 0,5-20 | |
| Structure^{®*2} XL | | | | 9 |
| Kationisches und/oder kationisierbares Tensid c) | 0,1-10 | | | |
| Esterquat und/oder Amidoamin | | 0,1-10 | 0,1-10 | |
| Varisoft^{®*3} EQ 100 | | | | 0,5 |
| Öl | | 0,01-5 | | |
| Citropol^{®*4} H | | | 0,01-2 | 0,5 |
| Aprikosenkernöl | | | 0,01-2 | 0,2 |
| Glycerin | | 0,1-5 | 0,1-5 | 1 |
| Wasser, Parfum, Konservierungsmittel; Zitronensäure bis pH 5,0 ± 0,5 | ad 100 | ad 100 | ad 100 | ad 100 |

| | | | | |
|---|---|---|---|---|
| ^{*1} INCI-Bezeichnung: Glycolipids; AS etwa 50%; Evonik ^{*2} INCI-Bezeichnung: Hydroxypropyl Starch Phosphate; National Starch ^{*3} INCI-Bezeichnung: Bis-(Isostearoyl/Oleyl Isopropyl) Dimonium Methosulfate; Evonik ^{*4} INCI-Bezeichnung: Polycitronellol; P2 Science | | | | |

Bei der Optimierung der erfindungsgemäßen Reinigungsgele wurden verschiedene Verdickungsmittel b) und verschiedene Pflegesubstanzen c) (kationische Polymere, weitere kationische Tenside, Silikone) eingesetzt und getestet.

Die besten Ergebnisse hinsichtlich einer optimalen Balance zwischen Stabilität und Performance (sehr gute Reinigungs- und Pflegeleistung) wurde mit der erfindungsgemäßen Wirkstoffkombination a), b), c) in einer wässrigen Gelmatrix erzielt.

Erfindungsgemäße Reinigungsgele sind außerdem besonders gut geeignet für die Behandlung gelockter und/oder krauser Haare, da ihre Applikation mit einer nur geringfügigen Verwirrung der Haare einhergeht und es somit nur zu einer äußerst geringen bis zu keiner Verkettung, Verklebung oder Verknotung der Haare kommt.

Das erfindungsgemäße Reinigungsgel 4 wurde in einem Halbseitentest an drei Modellen mit einem üblichen, schäumenden Pflegeshampoo (umfassend ein anionisches Sulfattensid, ein amphoteres Tensid sowie ein synthetisches kationisches Pflegepolymer und ein Silikon als Pflegesubstanzen) angewendet und beurteilt.

Die drei Modelle hatten unterschiedliche Haarfarben und Haarqualitäten.

Es wurden jeweils gleiche Mengen des erfindungsgemäßen Reinigungsgels 4 sowie der zuvor genannten Vergleichszusammensetzung auf die jeweiligen Kopfhälften aufgetragen und nach einer identischen Einwirkungszeit mit Wasser ausgespült.

Anschließend wurden die Eigenschaften der Haare von zwei Kopfhälften jeweils eines Kopfes direkt miteinander verglichen und dabei die Noten 1 - 6 vergeben (eine Beurteilung < 4 bedeutet, dass die jeweilige Anforderung nicht erfüllt wurde).

Aus den Benotungen wurden anschließend Mittelwerte gebildet.

Es wurden die folgenden Ergebnisse erzielt, wobei sich der zuerst angegebene Mittelwert jeweils auf die Beurteilung nach der Behandlung mit dem erfindungsgemäßen Reinigungsgel 4 bezieht:
a) Reinigung:
   - die Reinigungsleistung und die Ausspülbarkeit der beiden Zusammensetzungen wurden in etwa gleich bewertet (4,67 / 5 bzw. 5 /5)
b) Pflege:
   - Entwirrbarkeit (4,67 / 3,67) und Kämmbarkeit (5 / 4,33) nasser Haare wurden auf der mit dem erfindungsgemäßen Reinigungsgel behandelten Kopfhälfte signifikant besser bewertet; ebenso wie auf trockenen Haaren (Entwirrbarkeit: 4,5 / 3,5); Kämmbarkeit: 5 / 4,33)
   - Volumen (5 / 4,67) und Haarfülle (4,67 / 4,33) der mit dem erfindungsgemäßen Reinigungsgel behandelten Haare wurden leicht besser bewertet
   - die Gesamtbeurteilung der mit dem erfindungsgemäßen Reinigungsgel behandelten Haare war ebenfalls leicht besser (5 / 4,67)

## Patentansprüche

1. Kosmetische Reinigungszusammensetzung in Form eines wässrigen Gels, enthaltend - bezogen auf das Gesamtgewicht der Reinigungszusammensetzung -
a) 0,5 - 15 Gew.-% mindestens eines Glycolipids
b) 0,5 - 20 Gew.-% mindestens eines auf natürlichen Polymeren basierenden Verdickungsmittels
c) 0,1 - 10 Gew.-% mindestens eines biologisch abbaubaren kationischen oder kationisierbaren Tensids.

2. Kosmetische Reinigungszusammensetzung nach Anspruch 1, **dadurch gekennzeichnet, dass** das mindestens eine Glycolipid a) ausgewählt ist aus Rhamnolipiden, Sophorolipiden, Mannosylerythrollipiden, Trehaloselipiden, Cellobiolipiden, Saponinen oder Mischungen davon.

3. Kosmetische Reinigungszusammensetzung nach einem der Ansprüche 1 oder 2, **dadurch gekennzeichnet, dass** das mindestens eine Glycolipid a) ausgewählt ist aus Rhamnolipiden.

4. Kosmetische Reinigungszusammensetzung nach einem der Ansprüche 1 bis 3, **dadurch gekennzeichnet, dass** das mindestens eine auf natürlichen Polymeren basierende Verdickungsmittel b) ausgewählt ist aus Biopolymeren oder Derivaten von Biopolymeren, wie Gummen, Alginaten, Pektinen, Stärken oder Stärkederivaten, Cellulosen oder Cellulosederivaten, Dextrinen oder Mischungen davon.

5. Kosmetische Reinigungszusammensetzung nach einem der Ansprüche 1 bis 4, **dadurch gekennzeichnet, dass** das mindestens eine auf natürlichen Polymeren basierende Verdickungsmittel b) ausgewählt ist aus Stärken oder Stärkederivaten, vorzugsweise aus unter der INCI-Bezeichnung Hydroxypropyl Starch Phosphate bekannten Verbindungen.

6. Kosmetische Reinigungszusammensetzung nach einem der Ansprüche 1 bis 5, **dadurch gekennzeichnet, dass** das biologisch abbaubare kationische oder kationisierbare Tensid c) ausgewählt ist aus Esterquats, wie die unter den INCI-Bezeichnungen Distearoylethyl Hydroxyethylmonium Methosulfate, Distearoylethyl Dimonium Chloride, Distearoyl Dimonium Chloride, Distearoyl Dimonium Methosulfate, Bis-(Isostearoyl/Oleyl Isopropyl)Dimonium Methosulfate, Bis-(Isostearoyl/Oleyl Isopropyl)Dimonium Chloride bekannten Verbindungen sowie deren Mischungen.

7. Kosmetische Reinigungszusammensetzung nach einem der Ansprüche 1 bis 5, **dadurch gekennzeichnet, dass** das biologisch abbaubare kationische oder kationisierbare Tensid c) ausgewählt ist aus Amidoaminen, wie die unter den INCI-Bezeichnungen Brassicamidopropyl Dimethylamin, Stearamidopropyl Dimethylamin, Behenamidopropyl Dimethylamin, Cannabisamidopropyl Dimethylamine bekannten Verbindungen sowie deren Mischungen.

8. Kosmetische Reinigungszusammensetzung nach einem der Ansprüche 1 bis 7, enthaltend - bezogen auf das Gesamtgewicht der Reinigungszusammensetzung -
a) 0,5 - 15 Gew.-% mindestens eines Rhamnolipids
b) 0,5 - 20 Gew.-% mindestens einer Stärke oder Stärkederivats,
c) 0,1 - 10 Gew.-% mindestens eines Esterquats und/oder Amidoamins.

9. Kosmetische Reinigungszusammensetzung nach einem der Ansprüche 1 bis 8, enthaltend - bezogen auf das Gesamtgewicht der Reinigungszusammensetzung - 0,01 - 5 Gew.-% mindestens eines Öls, ausgewählt ist aus der Gruppe Polycitronellol, Polycitronellolacetat, Triolein oder Mischungen davon.

10. Kosmetische Reinigungszusammensetzung nach einem der Ansprüche 1 bis 9, enthaltend - bezogen auf das Gesamtgewicht der Reinigungszusammensetzung - 0,1 - 10 Gew.-% mindestens einer feuchtigkeitsspendenden Komponente, ausgewählt aus Propylenglycol, Butylenglycol, Glycerin, Sorbitol, Xylitol, Aloe Vera Gel, Hyaluronsäure(derivaten) oder Mischungen davon.

11. Kosmetische Reinigungszusammensetzung nach einem der Ansprüche 1 bis 10, enthaltend - bezogen auf das Gesamtgewicht der Reinigungszusammensetzung - 0,01 - 5 Gew.-% mindestens eines pflanzlichen Öls, ausgewählt aus Abyssinianöl, Amaranthsamenöl, Aprikosenkernöl, Arganöl, Avocadoöl, Babassuöl, Bambusöl, Baumwollsaatöl, Borretschsamenöl, Camelinaöl, Cranberrykernöl, Distelöl, Erdnussöl, Granatapfelkernöl, Grapefruitkernöl, Hanföl, Haselnussöl, Holundersamenöl, Johannesbeersamenöl, Jojobaöl, Kakaobutter, Kaktusfeigenöl, Krambesamenöl, Kürbiskernöl, Leinsamenöl, Macadamianussöl, Maiskeimöl, Malvenöl, Mandelöl, Mangokernöl, Marulaöl, Nachtkerzenöl, Olivenöl, Orangenöl, Palmöl, Pfirsichkernöl, Quinoaöl, Rambutanöl, Rapsöl, Reiskleieöl, Rizinusöl, Safloröl, Sanddornfruchtfleischöl, Sanddornkernöl, Sasanquanaöl, Sesamöl, Sheabutter, Sojaöl, Sonnenblumenöl, Teebaumöl, Traubenkernöl, Tsubakiöl, Walnussöl, Weizenkeimöl, Wiesenschaumkrautöl, Wildrosenöl, den flüssigen Anteilen von (süßem) Kokosöl oder Mischungen davon.

12. Kosmetische Reinigungszusammensetzung nach einem der Ansprüche 1 bis 11, enthaltend - bezogen auf das Gesamtgewicht der Reinigungszusammensetzung - mindestens 60 Gew.-% Wasser.

13. Kosmetische Reinigungszusammensetzung nach einem der Ansprüche 1 bis 12, **dadurch gekennzeichnet, dass** sie eine Viskosität im Bereich von 3,000 bis 30,000 mPas, mehr bevorzugt von 5,000 bis 30,000 mPas, besonders bevorzugt 10,000 bis 27,500 mPas und insbesondere 12,500 bis 25,000 mPas aufweist, gemessen bei 20°C mit einem Brookfield Viskosimeter; Spindel 6; 20s; 20 Upm.

14. Kosmetische Reinigungszusammensetzung nach einem der Ansprüche 1 bis 13, **dadurch gekennzeichnet, dass** sie einen pH-Wert im Bereich von 4,0 - 6,0 aufweist.

15. Verwendung einer kosmetischen Reinigungszusammensetzung nach einem der Ansprüche 1 bis 14 für die Reinigung und Pflege von Haaren, insbesondere zur Verbesserung der Entwirrbarkeits-, Kämmbarkeits- und der statischen Eigenschaften von Haaren sowie zur Verbesserung des Haarvolumens und der Haarfülle.
